# EUROPÄISCHE PATENTSCHRIFT

(11) **EP 1 648 526 B1**
(45) Veröffentlichungstag und Bekanntmachung des Hinweises auf die Patenterteilung: **09.09.2009**
(21) Anmeldenummer: 04741922.1
(22) Anmeldetag: 30.06.2004
(51) Int. Cl.: A61L 9/01, C07C 69/06

(54) **CIS-3,3,5-TRIMETHYLCYCLOHEXYLESTER**
CIS-3,3,5-TRIMETHYLCYCLOHEXYL ESTERS
ESTERS CIS-3,3,5-TRIMETHYLCYCLOHEXYLIQUES

(30) Priorität: 19.07.2003 DE 10332908
(43) Veröffentlichungstag der Anmeldung: 26.04.2006
(73) Patentinhaber: Symrise GmbH & Co. KG, 37603 Holzminden (DE)
(72) Erfinder: KUHN, Walter, 37603 Holzminden (DE); SURBURG, Horst, 37603 Holzminden (DE)
(74) Vertreter: Eisenführ, Speiser & Partner
(86) Internationale Anmeldenummer: PCT/EP2004/051292
(87) Internationale Veröffentlichungsnummer: WO 2005/009492

(56) Entgegenhaltungen:
- WO-A-01/43784
- DE-A- 2 026 409
- DE-A- 2 326 061
- DE-A- 2 406 849
- DE-A- 2 518 392

## Beschreibung

Die vorliegende Erfindung betrifft Gemische mit cis-3,3,5-Trimethylcyclohexylestern und trans-3,3,5-Trimethylcyclohexylestern, die Verwendung von cis-3,3,5-Trimethylcyclohexylestern als Riechstoffe sowie einzelne cis-3,3,5-Trimethylcyclohexylester und deren Verwendungen.

In der Parfümindustrie besteht grundsätzlich ein Bedarf an neuen Riechstoffen. Mit neuen Riechstoffen sollen neuartige Effekte erzielt, neue Modetrends kreiert und damit die steigende Nachfrage der Verbraucher nach neuen modernen Duftnoten befriedigt werden. Duftstoffe dieser Art sollen von ihrem Typ her möglichst originell sein und eine hohe sensorische Stärke und Ausgiebigkeit besitzen. Bereits in geringen Dosierungen sollen sich sensorisch spürbare, bemerkenswerte Dufteffekte erzielen lassen.

Besonders wertvoll sind dabei solche Riechstoffe, die in der Lage sind, Parfümkompositionen einen natumahen Geruch zu verleihen. Eine solche Natumähe kann beispielsweise durch die Verwendung von natürlich Extrakten wie Blütenextrakten und ätherischen Ölen erreicht werden. Naturprodukte wie natürliche Extrakte sind jedoch wegen der mühsamen Art ihrer Gewinnung teuer, nicht uneingeschränkt verfügbar und erheblichen Qualitätsschwankungen unterworfen. Dementsprechend besteht ein Bedarf an synthetisierbaren chemischen Stoffen und Gemischen mit einem möglichst natumahen Geruch.

Es ist bekannt, dass Isomerengemische von 3,3,5-Trimethylcyclohexylester Riechstoffe sind. So erwähnt S. Arctander, Perfume and Flavor Materials, Vol. I und II, Montclair, N. J., 1969, Eigenverlag, Monograph 3003, 3,3,5-Trimethylcyclohexylacetat als folgende Riechstoffeigenschaften aufweisend: schwach, süß-minzig, krautig. In dieser Veröffentlichung wird zwischen den Geruchseigenschaften von cis- und trans-3,3,5-Trimethylcyclohexylacetat nicht unterschieden.

In Tluszcze, Srodki Piorace, Kosmetyki 19, 516-20 (1975) sind die geruchlichen Eigenschaften des Formiats, Acetats und Propionats von 3,3,5-Trimethylcyclohexanol mit einem Gehalt von mehr als 95 % an trans-Isomeren beschrieben. Demnach soll das Formiat nach Kampher, Cineol, Iris und Sandelholz riechen. Das Acetat soll nach Waldkräutern riechen mit einem Thuja-, Lavendel- und Fichten-artigen Geruch, wobei dieser, Geruch als interessant und vorteilhaft für die Parfümerie beschrieben wird. Das Propionat soll einen Mango-artigen Geruch mit leichter Honignote und einem Iris-artigen Hintergrund besitzen.

In der JP 01 056798 sind Parfümöle für die Verwendung in bleichmittelhaltigen Waschmitteln beschrieben. Als eines der möglichen Parfümöle ist 3,5,5-Trimethylcyclohexylisobutyrat aufgeführt. Wiederum wird zwischen cis- und trans-Isomeren nicht unterschieden.

In der WO 01/43784 werden die Substanzen 3,3,5-Trimethylcyclohexylacetat, 3,3,5-Trimethylcyclohexylpropionat, 3,3,5-Trimethylcyclohexylcrotonat und 3,3,5-Trimethylcyclohexylbutyrat sowie deren Verwendung zum Neutralisieren von unangenehmen Gerüchen beschrieben. Die genannten Substanzen selbst sollen nur einen sehr geringen Eigengeruch besitzen. In der geruchlichen Bewertung wird dabei zwischen der cis- und trans-Isomeren dieser Substanzen nicht unterschieden.

DE 25 18 392 A betrifft neue Riechstoffe der Kohlensäure-alkyl-cycloalkylester der allgemei-nen Formel R₁O-CO-OR₂, deren Herstellung sowie diese enthaltene Riechstoffkompositionen. Diese Riechstoffe sollen eine sehr natürliche und komplexe Geruchsnote aufweisen.

DE 23 26 061 A betrifft Bis-(halogenphenoxy)-essigsäure-trimethylcyclohexylester, sowie Verfahren zu ihrer Herstellung und Arneimittel.

DE 2 406 849 A1 betrifft Nicotinsäure-trans-3,3,5-trimethylclyclohex-1-yl-ester, Verfahren zu seiner Herstellung und diesen Ester enthaltende Arzneimittel.

Aufgabe der vorliegenden Erfindung war es deshalb, weitere Riechstoffe anzugeben, die möglichst originell sind, eine hohe sensorische Stärke und Ausgiebigkeit besitzen und Parfümkompositionen einen möglichst naturnahen Geruch verleihen. Zudem sollten die Riechstoffe mit möglichst gleichbleibender Qualität und auf einfache Weise gewinnbar sein.

Die Suche nach geeigneten Riechstoffen, die zur vorliegenden Erfindung führte, wurde durch folgende Sachverhalte erschwert
- die Mechanismen der Geruchswahrnehmung sind nicht ausreichend bekannt,
- die Zusammenhänge zwischen der speziellen Geruchswahrnehmung einerseits und der chemischen Struktur des zugehörigen Riechstoffs andererseits nicht hinreichend erforscht,
- es gibt keinen Zusammenhang zwischen der Geruchscharakteristik und der toxikologischen Unbedenklichkeit einer Substanz,
- häufig bewirken bereits geringfügige Änderungen am strukturellen Aufbau eines bekannten Riechstoffs starke Änderungen der sensorischen Eigenschaften und beeinträchtigen die Verträglichkeit für den menschlichen Organismus.

Der Erfolg der Suche nach geeigneten Riechstoffen hängt deshalb stark von der Intuition des Suchenden ab.

Überraschenderweise hat sich nunmehr gezeigt, dass innerhalb der 3,3,5-Trimethylcyclohexylester die cis-3,3,5-Trimethylcyclohexylester besonders wertvolle Riechstoffe sind, wohingegen die trans-3,3,5-Trimethylcyclohexylester nur von geringem geruchlichen Wert sind, weil es ihnen an geruchlicher Intensität und Originalität fehlt.

Die Aufgabe wird erfindungsgemäß gelöst durch ein Gemisch gemäß Anspruch 1 umfassend einen oder mehrere cis-3,3,5-Trimethylcyclohexylester und einen oder mehrere trans-3,3,5-Trimethylcyclohexylester, wobei der Anteil an cis-3,3,5-Trimethylcyclohexylester den an trans-3,3,5-Trimethylcyclohexylester übersteigt.

Zwar ist auch in Tluszcze (siehe oben) angegeben, dass auch Gemische mit einem Anteil an trans-3,3,5-Trimethylcyclohexylester interessante geruchliche Eigenschaften besitzen; dies ist wohl auf den Restgehalt von 5 % cis-3,3,5-Trimethylcyclohexylester zurückzuführen. So hat beispielsweise das trans-3,3,5-Trimethylcyclohexylacetat nur eine vergleichsweise geringe geruchliche Intensität, wobei es nur relativ schwach esterartig und etwas fruchtig riecht und mit einer eher unangenehmen dumpfen, kampfrigen erdigen Note behaftet ist Im Gegensatz dazu zeigt das cis-3,3,5-Trimethylcyclohexylacetat einen strahlenden, frisch-fruchtigen, minzigen, kräuterartigen, leicht blumigrosigen Geruch. Dieser erhebliche Unterschied in der geruchlichen Bewertung war nicht vorhersehbar.

Cis-3,3,5-Trimethylcyclohexylester und diese(n) Ester enthaltende Gemische, insbesondere solche, bei denen der Anteil an cis-3,3,5-Trimethylcyclohexylester den Anteil an trans-3,3,5-Trimethylcyclohexylester übersteigt, sind entsprechend besonders geeignete Riechstoffe, Ihnen ist eine besondere geruchliche Sauberkeit und Klarheit eigen, so dass mit ihnen besonders gut neue, originelle und naturnahe Duftnoten kreiert werden können. Der Anteil an cis-3,3,5-Trimethylcyclohexylester in einem Gemisch sollte deshalb vorzugsweise über 80 % betragen, wobei ein Anteil von über 85 % und insbesondere ein Anteil von über 90 % aus den genannten Gründen besonders bevorzugt ist. Gemische mit einem hohen Anteil des besagten cis-Isomeren sind wegen ihrer Qualität und Ausgiebigkeit entsprechenden Isomeren-Gemischen mit einem niedrigen Anteil an cis-Isomeren deutlich hinsichtlich der Sauberkeit und Klarheit des Geruchseindrucks überlegen.

Besonders bevorzugt sind dabei solche Gemische, bei denen der Anteil an cis-3,3,5-Trimethylcyclohexylester zumindest doppelt so hoch ist wie der an trans-3,3,5-Trimethylcyclohexylester. Aufgrund dieses günstigen IsomerenVerhältnisses sind solche Gemische besonders geeignet zum Kreieren von neuen, originellen und natumahen Duftnoten.

Die cis- und trans-3,3,5-Trimethylcyclohexylester sind jeweils unabhängig voneinander ausgewählt aus der Gruppe bestehend aus 3,3,5-Trimethylcyclohexylformiat, 3,3,5-Trimethylcyclohexylacetat, 3,3,5-Trimethylcyclohexylpropionat, 3,3,5-timethylcyclohexylisobutyrat, 3,3,5-Trimethylcyclohexylbutyrat, 3,3,5-Trimethylcyclohexylcrotonat, 3,3,5-Trimethylcyclohexyltiglinat und 3,3,5-Trimethylcyclohexyl-3-methyl-2-butenoat, Den genannten cis-Isomeren sind jeweils besondere Duftnoten zu eigen, siehe dazu unten mehr. Cis-3,3,5-Trimethylcyclohexylester und insbesondere die genannten cis-Isomere sind deshalb allein, aber insbesondere auch gemischt mit dem jeweils entsprechenden trans-Isomer, besonders geeignet zum Kreieren neuer, origineller und natumaher Duftnoten.

Zur Lösung der Aufgabe ist es ferner vorteilhaft, wenn das Gemisch zumindest einen weiteren Riechstoff umfasst und so eine Riechstoffkomposition bildet. Auf diese Weise lassen sich besonders interessante und natürliche, aber auch neue und originelle Duftnoten kreieren. Riechstoffe, die zur Kombination ganz generell geeignet sind, finden sich z.B. in S. Arctander, Perfume and Flavor Materials, Vol. I und II, Montclair, N. J. 1969, Eigenverlag, oder K. Bauer et al., Common Fragrance and Flavor Materials, 4th Edition, Wiley-VCH, Weinheim 2001. Im einzelnen seien genannt

Extrakte aus natürlichen Rohstoffen wie Etherische Öle, Concretes, Absolues, Resine, Resinoide, Balsame, Tinkturen wie z,B. Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepresst; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur, Muskateller-Salbei-Öl; Muskatnussöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierte Inhaltsstoffe;

Einzel-Riechstoffe aus der Gruppe
der Kohlenwasserstoffe, wie z.B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z.B. Hexanol; Octanol; 3-Octanol; 2,6-Dimethylheptanol; 2-Methyl-2-heptanol; 2-Methyl-2-octanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z.B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd;1-(1-Methoxypropoxy)-(E/Z)-3-hexen;
der aliphatischen Ketone und deren Oxime wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
der aliphatischen schwefelhaltigen Verbindungen wie z.B. 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Undecensäurenitril; 2-Tridecensäurenitril; 3,12-Tridecadiensäurenitril; 3,7-Dimethyl-2,6-octadiensäurenitril; 3,7-Dimethyl-6-octensäurenitril;
der Ester von aliphatischen Carbonsäuren wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;4-Methyl-2-pentylcrotonat;
der acyclischen Terpenalkohole wie z.B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone wie z.B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetate von Geranial, Neral, 7-Hydroxy-3,7-dimethyloctanal;
der cyclischen Terpenalkohole wie z.B. Menthol; Isopulegol; alpha-Terpineol; Terpineol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
der cyclischen Terpenaldehyde und -ketone wie z. B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on;2-Methyl-4-(2,6,6-trimethyl-1-cyclohexen-1-yl)-2-butenal; Nootkaton; Dihydronootkaton; 4,6,8-Megastigmatrien-3-on; alpha-Sinensal; beta-Sinensal; acetyliertes Cedemholzöl (Methylcedrylketon);
der cyclischen Alkohole wie z.B. 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-ol;
der cycloaliphatischen Alkohole wie z.B. alpha,3,3-Trimethylcyclohexylmethanol;1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)-hexan-3-ocl;
der cyclischen und cycloaliphatischen Ether wie z.B. Cineol; Cedrylmethylether; Cyclododecylmethylether;1,1-Dimethoxycyclododecan; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3 -Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z.B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat, soweit das entsprechende Isomer nicht bereits im Gemisch enthalten ist; Decahydro-2-naphthylacetat; 2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. -6-indenylacetat; 4,7-Methano-3a,4,5,6,7,7a-hexahydro-5, bzw. -6-indenylpropionat; 4,7-Methano-3a,4,5,6,7,7a-hexahydro-5, bzw. -6-indenylisobutyrat; 4,7-Methanooctahydro-5, bzw. -6-indenylacetat;
der Ester cycloaliphatischer Alkohole wie z.B. 1-Cyclohexylethylcrotonat;
der Ester cycloaliphatischer Carbonsäuren wie z.B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether, 2-Phenylethylisoamylether, 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno-[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatische Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert-butylphenyl)propanal oder 2,4-Dimethyl-3-cyclohexencarbaldehyd; 6-Isopropylchinolin; 6-Isobutylchinolin; 6-sec.-Butylchinolin;2-(3-Phenylpropyl)pyridin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether, Thymol; Carvacrol; Diphenylether; beta-Naphthylmethylether, beta-Naphthylethylether, beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Besonders bevorzugt sind dabei Kombinationen des cis-3,3,5-Trimethylcyclohexylester-haltigen Gemisches bzw. dieser cis-Isomeren mit Riechstoffen, die eine frische und/oder fruchtige Note besitzen. Die genannten cis-Isomere können den Geruchseindruck solcher Riechstoffe besonders vorteilhaft ergänzen.

Im übrigen ist es bevorzugt, wenn das Gemisch bzw. die Riechstoffkomposition cis-3,3,5-Trimethylcyclohexylester in einer ausreichenden Menge enthält, um im Vergleich zu einem cis-3,3,5-Trimethylcyclohexylester-freien Gemisch mit ansonsten gleicher Zusammensetzung den Geruch des Gemisches in Richtung frischer und/oder fruchtiger Note zu verschieben. Der Anteil an cis-3,3,5-Trimethylcyclohexylester hängt dabei natürlich insbesondere von der Art und Konzentration der weiteren Gemischbestandteile ab. Häufig führt bereits ein geringer Anteil an cis-3,3,5-Trimethylcyclohexylester dazu, dass der Geruch des Gemischs im Vergleich zu einem entsprechenden Gemisch ohne das benannte cis-Isomer eine olfaktorisch spürbare frische und/oder fruchtige Duftnote erhält.

Entsprechend besonders bevorzugt sind solche Riechstoffkompositionen, bei denen der Anteil an cis-3,3,5-Trimethylcyclohexylester 0,1 bis 90 Gew.-%, insbesondere 0,5 bis 20 Gew.-%, besonders bevorzugt 2 bis 10 Gew.-% beträgt, bezogen auf die Gesamtmasse der Riechstoffkomposition.

Aufgrund ihrer Dufteigenschaften eignen sich folgende cis-3,3,5-Trimethylcyclohexylester besonders gut als Riechstoffe: cis-3,3,5-Trimethylcyclohexylformiat, cis-3,3,5-Trimethylcyclohexylacetat, cis-3,3,5-Trimethylcyclohexylpropionat, cis-3,3,5-Trimethylcyclohexylisobutyrat cis-3,3,5-Trimethylcyclohexylbutyrat, cis-3,3,5-Trimethylcyclohexylcrotonat, cis-3,3,5-Trimethylcyclohexyltiglinat und 3,3,5-Trimethylcyclohexyl-3-methyl-2-butenoat.

Cis-3,3,5-Trimethylcyclohexylester, Gemische oder Riechstoffkompositionen der oben beschriebenen Art enthaltend cis-3,3,5-Trimethylcyclohexylester eignen sich besonders zum Vermitteln, Modifizieren und/oder Verstärken einer frischen und/oder fruchtigen Geruchsnote.

Besonders geeignet zum Vermitteln, Modifizieren und/oder Verstärken eines frischen und/oder fruchtigen Geruchs in einer Parfumkomposition oder in einem parfümierten Erzeugnis ist es, das zu der Parfümkomposition bzw. zu dem parfümierten Erzeugnis eine sensorisch wirksame Menge eines cis-3,3,5-Trimethylcyclohexylesters bzw. eines Gemisches oder einer Riechstoffkomposition der oben beschriebenen Art gegeben wird. Auf diese Weise lassen sich die mit den cis-3,3,5-Trimethylcyclohexylestem bzw. den sie enthaltenen Gemischen der zuvor beschriebenen Art die oben genannten Vorteile verwirklichen.

Insbesondere bevorzugt ist die Verwendung von cis-3,3,5-Trimethylcyclohexylformiat zum Vermitteln, Modifizieren und/oder Verstärken eines Geruchs mit einer, mehreren oder sämtlichen der Noten frisch, terpenig, natürlich, nussig und minzig.

Ferner bevorzugt ist die Verwendung von cis-3,3,5-Trimethylcyclohexylacetat zum Vermitteln, Modifizieren und/oder Verstärken eines Geruchs mit einer, mehreren oder sämtlichen der Noten strahlend frisch-fruchtig, minzig, kräuterartig und blumig-rosig.

Daneben bevorzugt ist die Verwendung von cis-3,3,5-Trimethylcyclohexylpropionat zum Vermitteln, Modifizieren und/oder Verstärken eines Geruchs mit einer, mehreren oder sämtlichen der Noten frisch-fruchtig, terpenig und apfelig.

Zum Vermitteln, Modifizieren und/oder Verstärken eines Geruchs mit einer, mehreren oder sämtlichen der Noten fruchtig, frisch-holzig und ananasartig ist besonders gut cis-3,3,5-Trimethylcyclohexylisobutyrat verwendbar.

Cis-3,3,5-Trimethylcyclohexylbutyrat ist besonders gut verwendbar zum Vermitteln, Modifizieren und/oder Verstärken eines Geruchs mit einer, mehreren oder sämtlichen der Noten fruchtig und bananenartig.

Ebenfalls bevorzug ist die Verwendung von cis-3,3,5-Trimethylcyclohexyltiglinat zum Vermitteln, Modifizieren und/oder Verstärken eines Geruchs mit einer, mehreren oder sämtlichen der Noten fruchtig, pflaumig, holzig, Damascenon-artig

Zudem ist die Verwendung von cis-3,3,5-Trimethylcyclohexylcrotonat bevorzugt zum Vermitteln, Modifizieren und/oder Verstärken eines Geruchs mit einer, mehreren oder sämtlichen der Noten frisch, fruchtig, citrisch, pflaumig, Damascenon-artig.

Des weiteren ist die Verwendung von cis-3,3,5-Trimethylcyclohexyl-3-methyl-2-butenoat bevorzugt zum Vermitteln, Modifizieren und/oder Verstärken eines Geruchs mit einer, mehreren oder sämtlichen der Noten holzig, frisch, fruchtig, pflaumig.

Es versteht sich, dass auch zwei oder mehr cis-3,3,5-Trimethylcyclohexylester miteinander gemischt und als Riechstoff verwendet werden können, insbesondere zum Vermitteln, Modifizieren und/oder Verstärken einer frischen und/oder fruchtigen Note.

Die Erfindung betrifft ferner Verbindungen der allgemeinen Formel 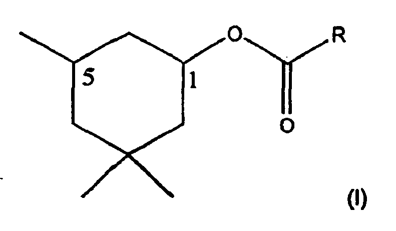 wobei C1 und C5 jeweils beide R- oder beide S-konfiguriert sind (cis-Isomer), und wobei R ein verzweigter oder unverzweigter Alkyl- oder Alkenylrest mit 4 oder mehr Kohlenstoffatomen ist.

Solche cis-3,3,5-Trimethylcyclohexylester eignen sich besonders gut zum Vermitteln, Modifizieren und/oder Verstärken eines frisch, fruchtig, pflaumig-Damascenon-artigen Geruchs.

Zu den cis-3,3,5-Trimethylcyclohexylestern gemäß Formel (I) zählen insbesondere cis-3,3,5-Trimethylcyclohexyltiglinat, cis-3,3,5-Trimethylcyclohexylcrotonat und cis-3,3,5-Trimethylcyclohexyl-3-methyl-2-butenoat. Nicht unter die Formel fällt cis-3,3,5-Trimethylcyclohexylbutyrat, das jedoch aufgrund seiner oben beschriebenen Verwendungsmöglichkeiten ebenfalls besonders geeignet ist zum Lösen der Aufgabe.

Zur Synthese der 3,3,5-Trimethylcyclohexylester mit einem hohen Gehalt an cis-Isomeren wird der Fachmann vorteilhaft von 3,3,5-Trimethylcyclohexanol mit einem entsprechend hohen Anteil des cis-Isomers ausgehen. Dieses ist durch Hydrierung von Isophoron (3,3,5-Trimethyl-2-cyclohexen-1-on) leicht zugänglich, wie z.B. in der Offenlegungsschrift DE 10160009 A1 beschrieben.

Die Herstellung der 3,3,5-Trimethylcyclohexylester erfolgt auf an sich bekannte Weise durch Umsetzung des 3,3,5-Trimethylcyclohexanols mit einer entsprechenden Carbonsäure, oder einem entsprechenden Carbonsäureanhydrid oder-halogenid.

Parfümöle, die cis-3,3,5-Trimethylcyclohexylester - insbesondere die oben beschriebenen erfindungsgemäßen Substanzen, Gemische und Riechstoffkompositionen - enthalten, können in flüssiger Form, unverdünnt oder mit einem Lösungsmittel verdünnt für Parfümierungen eingesetzt werden. Geeignete Lösungsmittel hierfür sind z.B. Ethanol, Isopropanol, Diethylenglykolmonoethyleter, Glycerin, Propylenglycol, 1,2-Butylenglycol, Dipropylenglycol, Diethylphthalat, Triethylcitrat, Isopropylmyristat usw.

Des weiteren können die Parfümöle, die die beiden erfindungsgemäßen 3,3,5-Trimethylcyclohexylester bzw. die oben beschriebenen Gemische oder Riechstoffkompositionen enthalten, an einem Trägerstoff adsorbiert sein, der sowohl für eine feine Verteilung der Riechstoffe im Produkt als auch für eine kontrollierte Freisetzung bei der Anwendung sorgt Derartige Träger können poröse anorganische Materialien wie Leichtsulfat, Kieselgele, Zeolithe, Gipse, Tone, Tongranulate, Gasbeton usw. oder organische Materialien wie Hölzer, Cellulose-basierende Stoffe, Zucker oder Kunststoffe wie PVC, Polyvinylacetate oder Polyurethane sein.

Parfümöle, die die beiden erfindungsgemäßen 3,3,5-Trimethylcyclohexylester bzw. die oben beschriebenen Gemische oder Riechstoffkompositionen enthalten, können auch mikroverkapselt, sprühgetrocknet, als Einschluss-Komplexe oder als Extrusions-Produkte vorliegen und in dieser Form dem zu parfümierenden Produkt hinzugefügt werden.

Gegebenenfalls können die Eigenschaften der derart modifizierten Parfümöle durch sog "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden.

Die Mikroverkapselung der Parfümöle kann beispielsweise durch das sogenannte Koazervationsverfahren mit Hilfe von Kapselmaterialien z.B. aus polyurethanartigen Stoffen oder Weichgelatine, erfolgen. Die sprühgetrockneten Parfümöle können beispielsweise durch Sprühtrocknung einer das Parfümöl enthaltenden Emulsion, bzw. Dispersion hergestellt werden, wobei als Trägerstoffe modifizierte Stärken, Proteine, Dextrin und pflanzliche Gummen verwendet werden können. Einschluss-Komplexe können z.B. durch Eintragen von Dispersionen von dem Parfümöl und Cyclodextrinen oder Harnstoffderivaten in ein geeignetes Lösungsmittel, z.B. Wasser, hergestellt werden. Extrusions-Produkte können durch Verschmelzen der Parfümöle mit einem geeigneten wachsartigen Stoff und durch Extrusion mit nachfolgender Erstarrung, ggf. in einem geeigneten Lösungsmittel, z.B. Isopropanol, erhalten werden.

Parfümöle, die die beiden erfindungsgemäßen 3,3,5-Trimethylcyclohexylester bzw. die oben beschriebenen Gemische oder Riechstoffkompositionen enthalten, können in konzentrierter Form, in Lösungen oder in oben beschriebener modifizierter Form verwendet werden für die Herstellung von z.B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigem, Fensterglasreinigem, Geschirrspülmittel, Bad- und Sanitärreinigern, Scheuermilch, festen und flüssigen WC-Reinigern, pulver- und schaumförmigen Teppichreinigem, Textilerfrischern, Bügelhilfen, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -Iotionen, Gesichtscremes und -Iotionen, Sonnenschutzcremes-und -Iotionen, After-suncremes und -Iotionen, Handcremes und -lotionen, Fußcremes und -Iotionen, Enthaarungscremes und -Iotionen, After-shave-Cremes und -Iotionen, Bräunungscremes und -Iotionen, Haarpflegeprodukten wie z.B. Haarsprays, Haargelen, festigenden Haarlotionen, Haarspülungen, permanenten und semipermanenten Haarfärbemitteln, Haarverformungsmitteln wie Kaltwellen und Haarglättungsmitteln, Haarwässern, Haarcremes und -Iotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien und Treibstoffen.

Die Erfindung wird nachfolgend anhand von Beispielen näher erläutert, ohne auf diese Beispiele beschränkt zu werden:

### Beispiel 1: Herstellung von 3,3,5-Trimethylcyclohexylacetat mit einem hohen Gehalt an cis-Isomeren

In einem 2 l Dreihalskolben mit aufgesetzter Destillationsvorrrichtung (20 cm Füllkörperkolonne mit Kolonnenkopf und gefüllt mit Glasringen) und Tropftrichter werden 568 g 3,3,5-Trimethylcyclohexanol mit einem cis-Gehalt von 90%, hergestellt nach DE 10160009 A1, vorgelegt. Nach Anlegen von einem schwachen Vakuum (ca. 600 mbar) wird auf eine Sumpftemperatur von ca. 140 °C erhitzt und langsam 612 g Essigsäureanhydrid zudosiert. Entstehende Essigsäure wird abdestilliert. Nach Beendigung der Dosierung wird der Ansatz noch 1 h bei der gleichen Temperatur gehalten. Im Anschluss wird fraktioniert destilliert Das erhaltene 3,3,5-Trimethylcyclohexylacetat siedet bei einer Kopftemperatur von ca. 85 °C bei 1 mbar Vakuum . Die Ausbeute beträgt 700g, entsprechend 95 %d. Th; der cis-Gehalt des so erhaltenen 3,3,5-Trimethylcyclohexylacetats liegt bei 90%.

Auf analoge Weise wird der entsprechende cis-3,3,5-Trimethylcyclohexylester der Ameisensäure hergestellt, wobei das gemischte Anhydrid aus Acetanhydrid und Ameisensäure eingesetzt wird.

### Beispiel 2: Herstellung weiterer Ester von 3,3,5-Trimethylcyclohexylacetat mit einem hohen Gehalt an cis-Isomeren

### Beispiel 2a: Cis- 3,3,5-Trimethylcyclohexylcrotonat

In einem 11-Reaktionskolben werden 71 g 3,3,5-Trimethylcyclohexanol mit einem cis-Gehalt von 90% zusammen mit 60 g Pyridin in 300 ml Methyl-tert-butylether vorgelegt und bei 0 bis 5°C während 30 min mit 63 g Crotonsäurechlorid versetzt. Man lässt das Reaktionsgemisch noch 1 h bei dieser Temperatur nachreagieren; dann lässt man auf Raumtemperatur erwärmen. Nach 1 h erwärmt man noch 30 min auf 50°C. Nach Abkühlen wird mit Wasser zerlegt, die organische Phase abgetrennt und erst mit verdünnter Salzsäure gewaschen und dann noch 30 min mit 5-proz. Natronlauge durchgerührt. Nach Neutralwaschen wird das Lösungsmittel abgezogen und der Rückstand über eine kurze Kolonne destilliert. Bei einem Sdp. Von 121°C bei 20 mbar werden 63 g reines 3,3,5-Trimethylcyclohexylcrotonat mit einem cis-Gehalt von 90%. MS: 210 (M*, 0,3), 195 (0,5), 141 (3), 124 (75), 109 (100), 95 (24), 87 (24), 82 (25), 69 (75), 55 (11), 41 (33).

Analog können die cis-3,3,5-Trimethylcyclohexylester der Propionsäure, Isobuttersäure und Buttersäure hergestellt werden.

### Beispiel 2b: Cis- 3,3,5-Trimethylcyclohexyltiglinat

71 g 3,3,5-Trimethylcyclohexanol mit einem cis-Gehalt von 90%, 60 g Tiglinsäure und 1 g p-Toluolsulfonsäure werden so lange am Wasserabscheider gekocht, bis keine merkliche Wasserscheidung mehr zu verzeichnen war. Die Reaktionslösung wird mit verdünnter Natronlauge entsäuert und mit Wasser neutral gewaschen. Nach Abziehen des Lösungsmittels wird der Rückstand über eine kurze Kolonne destilliert. Bei einem Sdp. Von 127-130°C bei 19 mbar werd 65 g reines 3,3,5-Trimethylcyclohexyltiglinat mit einem cis-Gehalt von 90% erhalten. MS: 224 (M+, 0,5), 209 (0,4), 141 (3), 124 (72), 109 (100), 101 (60), 95 (22), 83 (68), 69 (31), 55 (42), 41 (17).

### Beispiel 2c: Cis- 3,3,5-Trimethylcyclohexyl-3-methyl-2-butenoat

85,2 g 3,3,5-Trimethylcyclohexanol, 68,4 g Methyl-3-methyl-2-butenoat und 3 g Natriummethylat werden solange erwärmt, bis kein Methanol, das über eine kurze aufgesetzte Destillationskolonne abgenommen wird, mehr abgespalten wird. Nach Erkalten wird das Reaktionsgemisch mit Wasser zerlegt und Methyl-tert-butylether zugesetzt. Die organische Phase wird mit verdünnter Schwefelsäure, verdünnter Natronlauge und Wasser gewaschen und eingeengt. Der Rückstand wird über kurze Destillationskolonne fraktioniert. Bei einem Sdp. von 131°C bei 20 mbar werden 109 g reines 3,3,5-Trimethylcyclohexyl-3-methyl-2-butenoat mit einem cis-Gehalt von 90% erhalten. MS: 224 (M*, 0,3), 209 (0,2), 141 (6), 124 (32), 109 (49), 101 (60), 95 (12), 83 (100), 69 (36), 55 (28), 41 (15).

### Beispiel 3: Herstellen einer erfindungsgemäßen Riechstoffkomposition

Es wird eine Parfümbase durch Vermischen von folgenden synthetischen Riechstoffen hergestellt (alle Angaben in Gewichtsteilen):

| | |
|---|---|
| Benzylacetat | 30 |
| Ozonil* (2-Tridecennitril) 10% in Diethylphthalat | 5 |
| Dihydromyrcenol | 150 |
| Decanal | 1 |
| 2-Phenoxyethylisobutyrat | 100 |
| Methylcedrylketon | 35 |
| Hexylzimtaldehyd | 50 |
| Lilial^{®} | 30 |
| Linalylacetat | 100 |
| Galaxolide^{®} 50% in Diethylphthalat | 10 |
| Cedrylacetat | 30 |
| Zibeth Absolue synth. | 1 |
| Citronenterpene | 70 |
| Ethylvanillin | 3 |
| gamma-Undecalacton | 1 |
| Citronitril* (3-Methyl-5-phenyt-2-pentennitril) | 10 |
| Projasmon P* (2-Heptylcycloheptanon) | 1 |
| Agrumex HC* (2-tert.-Butylcyclohexylacetat) | 30 |
| Hexenylisobutyrat, cis-/trans- | 1 |
| Hexenylacetat, cis-/trans- | 1 |
| Limetteöl synth. | 10 |
| Diethylphthalat | 266 |
| Summe | 935 |

| | |
|---|---|
| * Handelsnamen der Fa. Symrise GmbH & Co KG, Holzminden, Germany | |

Durch die Zugabe von 65 Gewichtsteilen 3,3,5-Trimethylcyclohexylacetat mit einem Anteil an cis-Isomerem von 90% erhält diese Riechstoffkomposition einen überaus natürlichen grün-frischen, kräuterartigen und fruchtigen Charakter. Die Ausstrahlung und die Attraktivität werden deutlich wahrnehmbar erhöht.

Die Zugabe von 3,3,5-Trimethylcyclohexylacetat mit einem Anteil an cis-Isomerem von 90% verleiht der Mischung eine natürliche Ausstrahlung und unterstützt die frischen und fruchtigen Kopfnote.

## Patentansprüche

1. Gemisch, umfassend einen oder mehrere cis-3,3,5-Trimethylcyclohexylester und einen oder mehrere trans-3,3,5-Trimethylcyclohexylester, wobei der Anteil an cis-3,3,5-Trimethylcyclohexylester den an trans-3,3,5-Trimethylcyclohexylester übersteigt, wobei die cis- und trans-3,3,5-Trimethylcyolohexylester jeweils unabhängig voneinander ausgewählt sind aus der Gruppe bestehend aus 3,3,5-Trimethylcyclohexylformiat, 3,3,5-Trimethylcyclohexylacetat, 3,3,5-Trimethylcyclohexylpropionat, 3,3,5-Trimethylcyclohexylisobutyrat, 3,3,5-Trimethylcyclohexylbutyrat, 3,3,5-Trimethylcyclohexylcrotonat, 3,3,5-Trimethylcyclohexyltiglinat und 3,3,5-Trimethylcyclohexyl-3-methyl-2-butenoat.

2. Gemisch nach Anspruch 1, **dadurch gekennzeichnet, dass** der Anteil an cis-3,3,5-Trimethylcyclohexylester zumindest doppelt so hoch ist wie der an trans-3,3,5-Trimethylcyclohexylester.

3. Gemisch nach einem der vorherigen Ansprüche, enthaltend einen oder mehrere cis-3,3,5-Trimethylcyclohexylester in einer ausreichenden Menge, um im Vergleich zu einem cis-3,3,5-Trimethylcyclohexylester-freien Gemisch mit ansonsten gleicher Zusammensetzung den Geruch des Gemischs in Richtung frischer und/oder fruchtiger zu verschieben.

4. Verwendung eines cis-3,3,5-Trimethylcyclohexylesters zum Vermitteln, Modifizieren und/oder Verstärken eines Geruchs, wobei der cis-3,3,5-Trimethylcyclohexylester ausgewählt ist aus der Gruppe bestehend aus 3,3,5-Trimethylcyclohexylformiat, 3,3,5-Trimethylcyclohexylacetat, 3,3,5-Trimethylcyclohexylpropionat, 3,3,5-Trimethylcyclohexylisobutyrat, 3,3,5-Trimethylcyclohexylbutyrat, 3,3,5-Trimethylcyclohexylcrotonat, 3,3,5-Trimethylcyclohexyltiglinat und 3,3,5-Trimethylcyclohexyl-3-methyl-2-butenoat.

5. Verwendung von cis-3,3,5-Trimethylcyclohexylformiat nach Anspruch 4 zum Vermitteln, Modifizieren und/oder Verstärken eines Geruchs mit einer, mehreren oder sämtlichen der Noten frisch, terpenig, natürlich, nussig und minzig.

6. Verwendung von cis-3,3,5-Trimethylcyclohexylacetat nach Anspruch 4 zum Vermitteln, Modifizieren und/oder Verstärken eines Geruchs mit einer, mehreren oder sämtlichen der Noten strahlend frisch-fruchtig, minzig, kräuterartig und blumig-rosig.

7. Verwendung von cis-3,3,5-Trimethylcyclohexylpropionat nach Anspruch 4 zum Vermitteln. Modifizieren und/oder Verstärken eines Geruchs mit einer, mehreren oder sämtlichen der Noten frisch-fruchtig, terpenig, und apfelig.

8. Verwendung von cis-3,3,5-Trimethylcyclohexylisobutyrat nach Anspruch 4 zum Vermitteln, Modifizieren und/oder Verstärken eines Geruchs mit einer, mehreren oder sämtlichen der Noten fruchtig, frisch-holzig und ananasartig.

9. Verwendung von cis-3,3,5-Trimethylcyclohexylbutyrat nach Anspruch 4 zum Vermitteln, Modifizieren und/oder Verstärken eines Geruchs mit einer, mehreren oder sämtlichen der Noten fruchtig und bananenartig.

10. Verwendung von cis-3,3,5-Trimethylcyclohexyltiglinat nach Anspruch 4 zum Vermitteln, Modifizieren und/oder Verstärken eines Geruchs mit einer, mehreren oder sämtlichen der Noten fruchtig, pflaumig, holzig, Damascenon-artig.

11. Verwendung von cis-3,3,5-Trimethylcyclohexylcrotonat nach Anspruch 4 zum Vermitteln, Modifizieren und/oder Verstärken eines Geruchs mit einer, mehreren oder sämtliches der Noten frisch, fruchtig, citrisch, pflaumig, Damascenon-artig.

12. Verwendung von cis-3,3,5-Trimethylcyclohexyl-3-methyl-2-butenoat nach Anspruch 4 zum Vermitteln, Modifizieren und/oder Verstärken eines Geruchs mit einer, mehreren oder sämtlichen der Noten holzig, frisch, fruchtig, pflaumig.

13. Verbindung der allgemeinen Formel 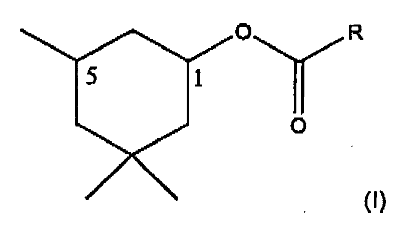 wobei C1 und C5 jeweils beide R- oder beide S-konfiguriert sind, und wobei R ein verzweigter oder unverzweigter Alkyl- oder Alkenylrest mit 4 oder mehr Kohlenstoffatomen ist.

14. Verbindung nach Anspruch 13, wobei die Verbindung ausgewählt ist aus der Gruppe bestehend aus cis-3,3,5-Trimethylcyclohexylbutyrat, cis-3,3,5-Trimethylcyclohexyltiglinat, cis-3,3,5-Trimethylcyclohexylcrotonat, cis-3,3,5-Trimethylcyclohexyl-3-methyl-2-butenoat.

## Claims

1. Mixture comprising one or more cis-3,3,5-trimethylcyclohexyl esters and one or more trans-3,3,5-trimethylcyclohexyl esters, wherein the content of cis-3,3,5-trimethylcyclohexyl esters exceeds that of trans-3,3,5-trimethylcyclohexyl esters, wherein the cis- and trans-3,3,5-trimethylcyclohexyl esters in each case independently of one another are chosen from the group consisting of 3,3,5-trimethylcyclohexyl formate, 3,3,5-trimethylcyclohexyl acetate, 3,3,5-trimethylcyclohexyl propionate, 3,3,5-trimethylcyclohexyl isobutyrate, 3,3,5-trimethylcyclohexyl butyrate, 3,3,5-trimethylcyclohexyl crotonate, 3,3,5-trimethylcyclohexyl tiglinate and 3,3,5-trimethylcyclohexyl 3-methyl-2-butenoate.

2. Mixture according to claim 1, **characterized in that** the content of cis-3,3,5-trimethylcyclohexyl esters is at least twice as high as that of trans-3,3,5-trimethylcyclohexyl esters.

3. Mixture according to one of the preceding claims, containing one or more cis-3,3,5-trimethylcyclohexyl esters in a sufficient amount to shift the smell of the mixture in the direction of fresher or fruitier compared with a cis-3,3,5-trimethylcyclohexyl ester-free mixture of otherwise the same composition.

4. Use of a cis-3,3,5-trimethylcyclohexyl ester for imparting, modifying and/or intensifying a smell, wherein the cis-3,3,5-trimethylcyclohexyl ester is chosen from the group consisting of 3,3,5-trimethylcyclohexyl formate, 3,3,5-trimethylcyclohexyl acetate, 3,3,5-trimethylcyclohexyl propionate, 3,3,5-trimethylcyclohexyl isobutyrate, 3,3,5-trimethylcyclohexyl butyrate, 3,3,5-trimethylcyclohexyl crotonate, 3,3,5-trimethylcyclohexyl tiglinate and 3,3,5-trimethylcyclohexyl 3-methyl-2-butenoate.

5. Use of cis-3,3,5-trimethylcyclohexyl formate according to claim 4 for imparting, modifying and/or intensifying a smell with one, several or all of the notes of fresh, terpene-like, natural, nutty and minty.

6. Use of cis-3,3,5-trimethylcyclohexyl acetate according to claim 4 for imparting, modifying and/or intensifying a smell with one, several or all of the notes of radiantly fresh-fruity, minty, herb-like and flowery-rosy.

7. Use of cis-3,3,5-trimethylcyclohexyl propionate according to claim 4 for imparting, modifying and/or intensifying a smell with one, several or all of the notes of fresh-fruity, terpene-like and apple-like.

8. Use of cis-3,3,5-trimethylcyclohexyl isobutyrate according to claim 4 for imparting, modifying and/or intensifying a smell with one, several or all of the notes of fruity, fresh-woody and banana-like.

9. Use of cis-3,3,5-trimethylcyclohexyl butyrate according to claim 4 for imparting, modifying and/or intensifying a smell with one, several or all of the notes of fruity and banana-like.

10. Use of cis-3,3,5-trimethylcyclohexyl tiglinate according to claim 4 for imparting, modifying and/or intensifying a smell with one, several or all of the notes of fruity, plum-like, woody and damascenone-like.

11. Use of cis-3,3,5-trimethylcyclohexyl crotonate according to claim 4 for imparting, modifying and/or intensifying a smell with one, several or all of the notes of fresh, fruity, citric, plum-like and damascenone-like.

12. Use of cis-3,3,5-trimethylcyclohexyl 3-methyl-2-butenoate according to claim 4 for imparting, modifying and/or intensifying a smell with one, several or all of the notes of woody, fresh, fruity and plum-like.

13. Compound of the general formula 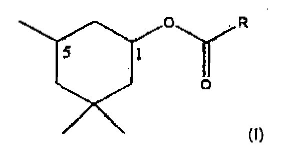 wherein C1 and C5 are in each case both in the R or both in the S configuration, and wherein R is a branched or unbranched alkyl or alkenyl radical having 4 or more carbon atoms.

14. Compound according to claim 13, wherein the compound is chosen from the group consisting of
cis-3,3,5-trimethylcyclohexyl butyrate,
cis-3,3,5-trimethylcyclohexyl tiglinate,
cis-3,3,5-trimethylcyclohexyl crotonate,
cis-3,3,5-trimethylcyclohexyl 3-methyl-2-butenoate.

## Revendications

1. Mélange comportant un ou plusieurs esters cis-3,3,5-triméthylcyclohexyliques et un ou plusieurs esters trans-3,3,5-triméthylcyclohexyliques, la proportion d'ester cis-3,3,5-triméthylcyclohexylique dépassant celle d'ester trans-3,3,5-triméthylcyclohexylique, les esters cis- et trans-3,3,5-triméthylcyclohexyliques, respectivement indépendamment l'un de l'autre, étant choisis parmi le groupe consistant en le formiate de 3,3,5-triméthylcyclohexyle, l'acétate de 3,3,5-triméthylcyclohexyle, le propionate de 3,3,5-triméthylcyclohexyle, l'isobutyrate de 3,3,5-triméthylcyclohexyle, le butyrate de 3,3,5-triméthylcyclohexyle, le crotonate de 3,3,5-triméthylcyclohexyle, le tiglinate de 3,3,5-triméthylcyclohexyle et le 3-méthyl-2-buténoate de 3,3,5-triméthylcyclohexyle.

2. Mélange selon la revendication 1, **caractérisé en ce que** la proportion d'ester cis-3,3,5-triméthylcyclohexylique est au moins deux fois plus élevée que celle d'ester trans-3,3,5-triméthylcyclohexylique.

3. Mélange selon l'une quelconque des revendications précédentes, contenant un ou plusieurs esters cis-3,3,5-triméthylcyclohexyliques en une quantité suffisante pour, en comparaison d'un mélange exempt d'ester cis-3,3,5-triméthylcyclohexylique avec la même composition par ailleurs, déplacer l'odeur du mélange dans la direction « plus frais » et/ou « plus fruité ».

4. Utilisation d'un ester cis-3,3,5-triméthylcyclohexylique pour la transmission, la modification et/ou le renforcement d'une odeur, sachant que l'ester cis-3,3,5-triméthylcyclohexylique est choisi parmi le groupe consistant en le formiate de 3,3,5-triméthylcyclohexyle, l'acétate de 3,3,5-triméthylcyclohexyle, le propionate de 3,3,5-triméthylcyclohexyle, l'isobutyrate de 3,3,5-triméthylcyclohexyle, le butyrate de 3,3,5-triméthylcyclohexyle, le crotonate de 3,3,5-triméthylcyclohexyle, le tiglinatc de 3,3,5-triméthylcyclohexyle et le 3-méthyl-2-buténoate de 3,3,5-triméthylcyclohexyle.

5. Utilisation du formiate de 3,3,5-triméthylcyclohexyle selon la revendication 4 pour la transmission, la modification et/ou le renforcement d'une odeur avec une ou plusieurs des notes ou avec toutes les notes frais, terpénique, naturel, de noix et de menthe.

6. Utilisation de l'acétate de 3,3,5-triméthylcyclohexyle selon la revendication 4 pour la transmission, la modification et/ou le renforcement d'une odeur avec une ou plusieurs des notes ou avec toutes les notes frais-fruité éclatant, de menthe, de fines herbes et de fleurs/de roses.

7. Utilisation du propionate de 3,3,5-triméthylcyclohexyle selon la revendication 4 pour la transmission, la modification et/ou le renforcement d'une odeur avec une ou plusieurs des notes ou avec toutes les notes frais-fruité, terpénique et de pomme.

8. Utilisation de l'isobutyrate de 3,3,5-triméthylcyclohexyle selon la revendication 4 pour la transmission, la modification et/ou le renforcement d'une odeur avec une ou plusieurs des notes ou avec toutes les notes fruité, boisé frais et d'ananas.

9. Utilisation du butyrate de 3,3,5-triméthylcyclohexyle selon la revendication 4 pour la transmission, la modification et/ou le renforcement d'une odeur avec une ou plusieurs des notes ou avec toutes les notes fruité et de banane.

10. Utilisation du tiglinate de 3,3,5-triméthylcyclohexyle selon la revendication 4 pour la transmission, la modification et/ou le renforcement d'une odeur avec une ou plusieurs des notes ou avec toutes les notes fruité, de prune, boisé, de damascénone.

11. Utilisation du crotonate de 3,3,5-triméthylcyclohexyle selon la revendication 4 pour la transmission, la modification et/ou le renforcement d'une odeur avec une ou plusieurs des notes ou avec toutes les notes frais, fruité, citroné, de prune, de damascénone.

12. Utilisation du 3-méthyl-2-buténoate de 3,3,5-triméthylcyclohexyle selon la revendication 4 pour la transmission, la modification et/ou le renforcement d'une odeur avec une ou plusieurs des notes ou avec toutes les notes boisé, frais, fruité, de prune.

13. Composé de la formule générale 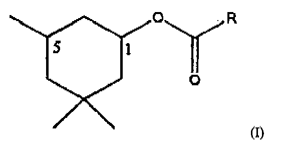 dans laquelle C1 et C5 ont tous deux la configuration R ou tous deux la configuration S, et dans laquelle R est un radical alkyle ou alcényle ramifié ou non ramifié avec 4 atomes de carbone ou plus.

14. Composé selon la revendication 13, le composé étant choisi parmi le groupe consistant en
le butyrate de cis-3,3,5-triméthylcyclohexyle
le tiglinate de cis-3,3,5-triméthylcyclohexyle
le crotonate de cis-3,3,5-triméthylcyclohexyle
le 3-méthyl-2-buténoate de cis-3,3,5-triméthylcyclohexyle.
